# EUROPÄISCHE PATENTSCHRIFT

(11) **EP 0 883 812 B1**
(45) Veröffentlichungstag und Bekanntmachung des Hinweises auf die Patenterteilung: **20.06.2001**
(21) Anmeldenummer: 97907002.6
(22) Anmeldetag: 09.01.1997
(51) Int. Cl.: G01N 33/547, G01N 33/548

(54) **(BIO)CHEMISCHE REAGENZ-FESTPHASEN, VERFAHREN ZU DEREN HERSTELLUNG SOWIE DEREN VERWENDUNGEN**
(BIO)CHEMICAL REAGENT SOLID PHASES, PROCESS FOR THEIR PRODUCTION AND THEIR APPLICATIONS
PHASES SOLIDES DE REACTIFS (BIO)CHIMIQUES, LEUR PROCEDE DE FABRICATION ET LEURS UTILISATIONS

(30) Priorität: 12.01.1996 DE 19600930
(43) Veröffentlichungstag der Anmeldung: 16.12.1998
(60) Teilanmeldung: 00116474.8
(73) Patentinhaber: FORSCHUNGSZENTRUM JÜLICH GMBH, 52425 Jülich (DE)
(72) Erfinder: BERLIN, Peter, D-52428 Jülich (DE); TILLER, Jörg, D-07749 Jena (DE); KLEMM, Dieter, D-99425 Weimar (DE)
(86) Internationale Anmeldenummer: DE9700072
(87) Internationale Veröffentlichungsnummer: WO9725621

(56) Entgegenhaltungen:
- EP-A- 0 131 369
- DE-C- 473 097
- US-A- 5 015 387

## Beschreibung

Die Erfindung betrifft (bio)chemische Reagenz-Festphasen, die aus einer polymeren oder makromolekularen, NH₂-haltigen Trägerverbindung bestehen, an die Kopplungsstrukturen über NH₂-Gruppen kovalent gebunden sind, welche weiterhin mit einer Rezeptor- und/oder Indikatorverbindung verknüpft sind und die somit die Rezeptor- und/oder Indikatorverbindungen an den Träger koppeln. Die Erfindung betrifft weiterhin ein Verfahren zur Herstellung von (bio)chemischen Reagenz-Festphasen sowie deren Verwendungen.

Bei den (bio)chemischen Reagenz-Festphasen der genannten Art werden durch die Kopplung von Rezeptorverbindungen an eine Trägerverbindung die Rezeptoren immobilisiert. Die Anwendung solcher immobilisierter Rezeptorverbindungen ist auf verschiedenen Gebieten seit längerer Zeit bekannt. Typische Einsatzgebiete sind die Biotechnologie, die (bio)chemische Sensorik und die biochemische Analytik, wie z.B. die Chromatographie. Von Interesse sind insbesondere immobilisierte Biover-bindungen, wie Enzyme oder Immunoproteine. Bei diesen wird die (Enzym-)Katalysefunktion bzw. Biokomplementarität zur Herstellung und/oder Reinigung von Substanzen genutzt, oder die Biokomplementarität wird für Analyt-Stoff-Erkennungs- und Signalprozesse analytisch eingesetzt.

Bezüglich der Immobilisierungsverfahren und der Zusammensetzung von Reagenz-Festphasen findet man in der Fachliteratur eine enorme Varianten-Vielfalt. Sie ergibt sich vor allem durch die Modifizierung
- der Struktur der Trägerverbindungen, wie z.B
   * makromolekularer Träger organischer und anorganischer Natur, wie Collagene, Dextrane bzw. poröse Gläser u.a. und
   * polymerer Träger polarer und unpolarer Natur, wie Polyamide, Polyurethane bzw. Polystyrole, Polyethylene u.a.
- der Immobiliesierungsart bzw. -verfahren, wie z.B.
   * Immersion oder Adsorption
   * elektrostatische oder kovalente Fixierung, z.B. via sog. bifunktioneller Kopplungsverbindung, typischerweise mittels Glutardialdehyd u.v.a., oder
   * Anwendung üblicher Synthesereaktionen, z.B. Substitutionsreaktionen, Diazotierungsreaktion u.a.
- der Rezeptorverbindung, z.B.
   * Bioverbindungen, wie Enzyme (Oxidoreduktasen, Proteasen u.a.) oder Immunoproteine (Antikörper) oder
   * organische Rezeptorverbindungen, wie Kronenäther für Ionen u.a.
   (vgl. z.B. Hermanson, G.T. et al. (Ed.): Immobilized Affinity Ligand Technics, Academic Press, 1992 oder Chibatu, I. (Ed.): Immobilized enzymes, research and development, Kodanska Scientific Books, 1978).

Insbesondere für die Immobilisierung von Bioverbindungen, wie von Enzymen oder Immunoproteinen, werden als Trägerverbindung auch Polysaccharide, insbesondere Cellulose, angewendet. Dabei findet man zu allen wesentlichen Immobilisierungskategorien, wie
- Carrier-Einschluß,
- Ionen-Beziehung und
- kovalente Bindung
eine Varianten-Vielfalt, auch bezüglich der einbezogenen Biover-bindungen. In Tabelle 1 ist eine Beispiel-Auswahl zusammen-gestellt (vgl. wie oben zitiert Hermanson, G.T. et al.):

**Tabelle 1:**

| Beispiele für Enzym-Immobilisierungen an Cellulosederivaten | | |
|---|---|---|
| **Cellulosederivat** | **Immobilisierungs-Typ** | **Enzym-Beispiel** |
| Butylacetatcellulose | Carrier-Einschluß | Urease u.a. |
| Nitrocellulose | Carrier-Enschluß | Lactase, Asparaginase u.a. |
| Kollodium | Carrier-Einschluß | Lactase, Urease u.a. |
| DEAE-Cellulose | Ionen-Beziehung | Katalase, Invertase, Pepsin u.a. |
| TEAE-Cellulose | Ionen-Beziehung | Aspartase u.a. |

| *kovalente Bindung an:* | | |
|---|---|---|
| p-Aminobenzylcellulose | Diazo-Reaktion | Katalase, Invertase, Pepsin, Aspartase u.a. |
| p-Aminobenzoylcellulose | Diazo-Reaktion | Trypsin, Chymotrypsin u.a. |
| Bromcyan-aktivierte Cellulose | Bromcyan-Aktivierung | Xanthin-Oxidase, Lactase, Dextranase u.a. |
| CM-Cellulose, AE-Cellulose | Carbodiimid-Methode | Peroxidase u.a. |
| Nitrocellulose-Membranen | Membran-Einschluß | Glucose-Oxidase, Peroxidase u.a. |
| Bromcyan-aktivierte Cellulose-Membranen | Membran-Einschluß/ Bromcyan-Aktivierung | Lactase, Trypsin u.a. |

Die Reihe in Tabelle 1 ließe sich sowohl hinsichtlich weiterer Cellulosederivate, z.B. Cellulosecarbonate, Chloracetylcellulose, Bromacetylcellulose u.v.m., als auch in Bezug auf weitere Bioverbindungen, z.B. weitere Enzymspezies, NAD- und Pyridoxal-phosphat-Coenzym, Vitamin B₁₂, Immunoproteine u.v.m., fortsetzen. Im Vordergrund der genannten Beispiele stehen präparative oder analytische, z.B. chromatographische, Anwendungen, bei denen es ohne besondere molekülgeometrische Anforderungen lediglich auf die Beladungsdichte der Trägerverbindung mit Rezeptorverbindung bzw. Enzym ankommt.

In Bezug auf die funktionellen Anforderungen werden beispielsweise an die Enzym-Tmmobilisierungsverfahren je nach Einsatzgebiet sehr unterschiedliche Maßstäbe angelegt. Zielkriterien einer effektiven Enzym-Immobilisierung sind
- eine präzise Faltung des Proteins
- eine freie Substrat-Zugänglichkeit zum aktiven Zentrum
- ein effektiver Produktabgang
- die Trägerfixierung an der Enzym-MolekülPeripherie
- eine hohe Enzym-Beladungsdichte pro Trägermatrix-Oberfläche und
- im Falle einer Anwendung in der Sensorik: eine Trägermatrix mit Signalstruktur-Merkmalen, die eine maximale optische bzw. Elektronen-Transfer-Signalübertragung gestatten.

Die Anwendung von Reagenz-Festphasen in der (bio)chemischen Sensorik erfordert es, daß bei jeder Sensor-Entwicklung für den jeweiligen Analyt eine neue Strukturoptimierung nach den oben angeführten Kriterien erfolgen muß; denn die Sensor-Lösung für einen Analyten läßt sich kaum auf andere Analyte ohne weiteres übertragen. Der diesbezüglich noch mängelbehaftete Entwicklungsstand auf dem (bio)chemischen Sensorgebiet läßt den Einsatz vieler wissenschaftlicher Sensor-Entwicklungen unter Praxisbedingungen häufig scheitern, weil zwischen den Ansprüchen der Sensor-Anwender und der meist limitierten Funktionsstabilität und den Querempfindlichkeiten der Sensoren noch eine große Lücke klafft.

Eine Lösung des Problems lassen Sensor-Transducer erwarten, die auf "maßgeschneiderten", supramolekularen Architekturen mit ver-besserten Signal-Übertragungseigenschaften basieren. Es sind Bei-spiele aus der Enzymelektroden-Entwicklung bekannt, die auf polymeren Trägerverbindungen mit sog. Elektronen-Mediatorstrukturen, z.B. Ferrocenderivaten, und immobilisierten Enzymen basieren, um eine verbesserte Signal-Transduktion durch Verminderung der Elektronen-Übergangs-"Barrieren" und der Meßelektrode zu erzielen (vgl. z.B. Frew, J.E. und Hill, H.A.O. (1987): Electrochemical Biosensors, Anal. Chem. 59, 933A; Frew, J.E. und Hill, H.A.O. (1987): Electron-transfer-Biosensors, Phil. Trans. R. Soc. B316, 95; Bockris, J.O'M. und Khan, S.U.M. (Ed.): Surface Electro-chemistry - A Molecular Level Approach, Plenum Press, 1993).

Erheblich komplexere supramolekulare Architekturen sind im Falle (bio)chemischer Glasfasersensor-Transducer erforderlich. Die höhere Komplexizität ist darin begründet, daß zum einen das funk-tionsfähige Enzym-Protein und zum anderen in den meisten Fällen eine zusätzliche Strukturkomponente, z.B. eine Indikatorstruktur, - in molekülgeometrisch ausgezeichneter Positionierung der Strukturen zueinander - an der polymeren oder makromolekularen Trägermatrix vorliegen muß. Ausnahmen, bei denen eine zusätzliche Signalstruktur nicht erforderlich ist, bilden Analyt-Erkennungs-strukturen, die beides - die Analyt-Erkennung und die optische Signal-Übertragung - Struktur-inherent bewirken; dafür gibt es bisher aber nur wenige Beispiele.

Desweiteren werden auf dem Gebiet der Glasfasersensorik bereits Reagenz-Festphasen mit Cellulosederivaten als Trägerverbindung eingesetzt, an denen via Vinylsulfonyl-Gruppen als Koppler pH-Indikatoren immobilisiert werden (Weigl, B.H. et al. (1993): Robust carbon dioxid optrode based on a covalently immobilized pH-indicator, SPIE-Vol. 2068, 2). Auch wird der Einsatz von Celluloseacetat-Membranen in Kombination mit pH-Indikatoren beschrieben (Sansubrino, A. und Mascini, M. (1994): Development of an optical fiber sensor for ammonia, urea, urease and IgG, Biosens. Bioelectron. 9, 207).

Die Entwicklung (bio)chemischer Glasfasersensoren ist von besonderem Interesse, weil sie auf Grund ihrer Integrationsfähig-keit, der Möglichkeit eines hohen Miniaturisierungsgrades und weiterer vorteilhafter Eigenschaften gut geeignet sind, in analytischen Mikrosystemen, z.B. in der medizinischen in vivo-Diagnostik, eingesetzt zu werden. Eine wesentliche, bisher aber noch fehlende Voraussetzung dafür sind Sensor-Transducer mit verbesserten Signal-Übertragungseigenschaften auf der Grundlage entsprechend "maßgeschneiderter" supramolekularer Architekturen, die sich aus leicht zugänglichen, möglichst preiswerten Ausgangs-stoffen und nach einer möglichst einfachen Verfahrensweise herstellen lassen.

Die EP-A-0131369 zeigt eine Immobilisierung von physiologisch aktiven Substanzen an Cellulose-Fiber mittels polymerer Carrierverbindungen, die eine Säure-Anhydrid-Funktion haben. Als physiologisch aktive Substanzen werden eine Vielzahl von z.B. Enzymen, Immunoproteinen, Hormonen u.a., aufgezählt.

Die Cellulose-Fiber unterschiedlicher Länge werden mit einer Lösung von Poly(Carboxyl-Anhydriden), z.B. Poly(Maleinsäure-Anhydrid), Poly(Acrylsäure-Anhydrid) u.a., bei unterschiedlichen Temperaturen und nach unterschiedlicher Behandlungsdauer aktiviert. Die behandelten Cellulose-Fiber werden mit der physiologisch aktiven Substanz in einer Pufferlösung in Kontakt gebracht. Danach liegt die physiologisch aktive Substanz an Cellulose-Fiber immobilisiert vor. Sie werden z.B. als Katalysatoren chemischer Reaktionen, in der biochemischen Praxis oder als medizinische Materialien eingesetzt.

Es ist Aufgabe der Erfindung, neue (bio)chemische Reagenz-Festphasen bereit zu stellen, deren Architektur möglichst breit-gefächerte Modifizierungsmöglichkeiten bietet und die daher viel-seitig einsetzbar sind, insbesondere als analyt-sensitive Festphasen in der Sensorik. Es ist ferner Aufgabe der Erfindung, ein einfaches Verfahren zur Herstellung derartiger Reagenz-Festphasen zu schaffen.

Die Aufgabe wird erfindungsgemäß dadurch gelöst, daß an einer polymeren oder makromolekularen, NH₂-haltigen Trägerverbindung Kopplungsstrukturen über NH₂-Gruppen kovalent gebunden sind, wobei die Kopplungsstrukturen aus Ascorbinsäure oder Dehydroascorbinsäure oder einer diesen Substanzen strukturell ähnlichen Verbindung bestehen. Die Kopplungsstrukturen sind weiterhin mit einer Rezeptor- und/oder Indikatorverbindung verknüpft, so daß diese an den Träger gekoppelt bzw. immobilisiert sind. Als der Ascorbin-säure oder Dehydroascorbinsäure strukturell ähnliche Substanz kommt insbesondere L-2,3-Diketogulonsäure oder ein Derivat davon in Betracht. Auch können als strukturell ähnliche Verbindungen beispielsweise Verbindungen mit mindestens zwei Ketogruppierungen, wie Acetylaceton, zum Einsatz kommen.

In einer bevorzugten Ausführungsform wird als Träger-verbindung ein Cellulosederivat der allgemeinen Formel I eingesetzt, 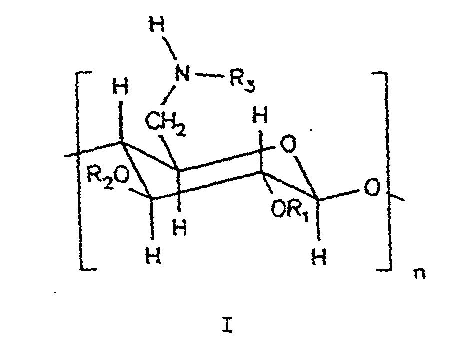 in dem
R₁, R₂ = H
oder R₁ = H, R₂ = Substituent mit einem Substitutionsgrad ≤ 1
oder R₁ = Substituent mit einem Substitutionsgrad < 1, R₂ = H
oder R₁, R₂ = Substituent mit einem Substitutionsgrad ≤ 2
und R₃ ein aromatischer Substituent, der mindestens eine freie Aminogruppe enthält und einem Substitutionsgrad ≤ 1 bedeutet.

Der Rest R₃ ist vorzugsweise ein Aminrest der Formel 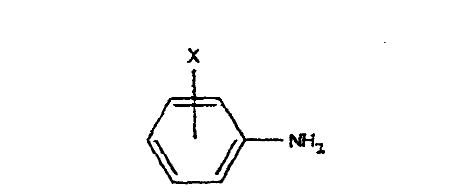 X= H oder H und/oder Substituenten

Als Amine bzw. Aminreste können aber auch beispielsweise Verbindungen mit den folgenden Strukturformeln vertreten sein: 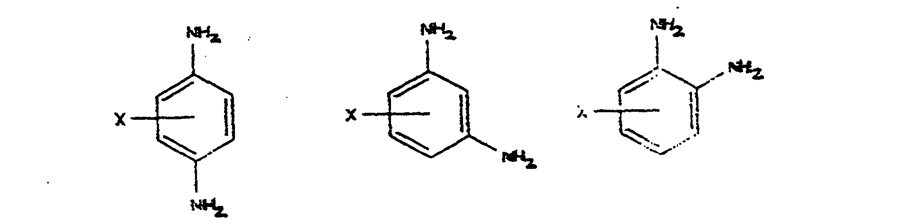 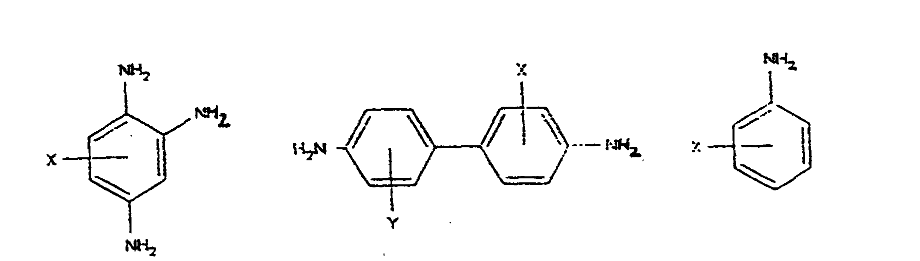 X = H oder H und/oder Substituenten
Y = H oder H und/oder Substituenten

Gemäß der Formel I ist das Grundgerüst entweder Cellulose (R₁, R₂ = H) oder ein Cellulosederivat. In Fällen, in denen ein Cellulose-derivat-Grundgerüst vorliegt, ist der Substituent in R₁- und/oder R₂-Position ein Alkyl, vorzugsweise CH₃, oder Acyl, vorzugsweise Acetyl, oder ein Tosyl- oder Tresylrest. Dabei kann der Substituent in den genannten Positionen ausschließlich ein Substituent der genannten Art sein, oder es können in der R₁- und/oder R₂-Position innerhalb eines Makromoleküls auch unterschiedliche Substituenten der genannten Art vertreten sein.

Die eine solche Trägerverbindung enthaltenden (bio)chemischen Reagenz-Festphasen sind insbesondere als analyt-sensitive Festphasen in der Sensorik hervorragend geeignet, da die Trägerverbindungen vor allem Redox-chromogene Eigenschaften haben, die durch vielfältige Strukturabwandlungen anhand bekannter Kupplungsreaktionen an den freien aromatischen NH₂-Gruppierungen breitgefächerte chromogene Modifikationsmöglichkeiten gestatten. Aufgrund dieser Eigenschaften können supramolekulare Architekturen bereit gestellt werden, die bei "Erkennung" des Analyten, (d.h. wenn die zum Rezeptor affine Verbindung mit diesem in Wechselwirkung tritt), dies optisch bzw. durch Elektronen-Transfer "signalisieren".

Die erfindungsgemäßen supramolekularen "Basis"-Architekturen bieten nunmehr die folgenden Erweiterungs- bzw. Modifizierungsmöglichkeiten:

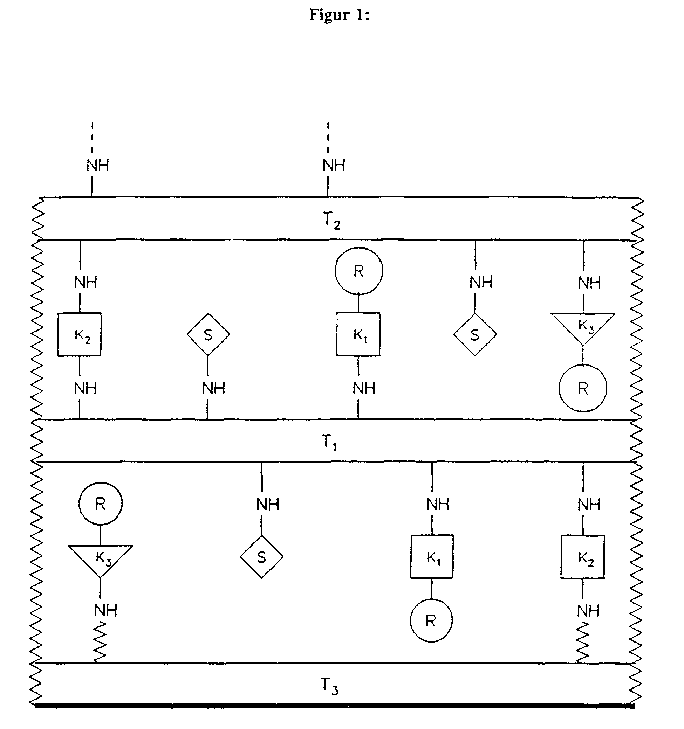

Es kann über mindestens einer Kopplungsstruktur wenigstens eine weitere, polymere und/oder makromolekulare, NH₂-haltige Träger-verbindung nach dem "sandwich-Prinzip" geknüpft sein, wobei an NH₂-Gruppen jeder weiteren Trägerverbindung wiederum Rezeptorverbindungen durch weitere Kopplungsstrukturen fixiert sein können. Zur Veranschaulichung ist eine solche mögliche Modifikation in der Figur 1 schematisch dargestellt und wird im folgenden näher erläutert:

In dem Schaubild ist eine Trägermatrix T₁ über NH₂-Gruppen mit Kopplungsstrukturen K verknüpft, wobei die erfindungsgemäßen Kopplungsstrukturen als Quadrate K₁ und K₂ dargestellt sind. An den Träger T₁ sind über Kopplungsstrukturen K₁ Rezeptorverbindungen R immobilisiert. Weitere Kopplungsstrukturen K₂ des Trägers T₁ sind über NH₂-Gruppen mit den Trägerverbindungen T₂ und T₃ nach dem "sandwich-Prinzip" verknüpft. An den Trägern T₂ und T₃ sind über weitere NH₂-Gruppen via Kopplungsstrukturen K₃ weitere Rezeptoren immobilisiert. Bei den Kopplungsstrukturen K₃ kann es sich ebenfalls um erfindungsgemäße Kopplungsverbindungen handeln, oder es können andere NH₂-reaktive homo- bzw. hetero- bifunktionelle oder multifunktionelle Kopplungsverbindungen bzw. sog. "Cross-Linker" eingesetzt werden. Beispielsweise können angewendet werden:
- Cyanurchlorid oder
- Dialdehyde, wie Glutardialdehyd, oder
- Diketone, wie Acetylaceton, oder
- Verbindungen, die mindestens eine Aldehyd- und mindestens eine Ketogruppierung enthalten oder Verbindungen mit mehr als zwei Aldehyd- und/oder Ketogruppierungen oder
- p-chinoide Verbindungen, wie Benzochinon, oder
- aromatische Disulfonsäure-dichloride, wie Benzol-1,4-disulfonsäure-dichlorid, oder
- aktivierte Diesterverbindungen, wie Bissuccinimidyl- und/oder Bis-maleimidyl-dicarbonsäurediester, oder
- Bis-diazoverbindungen, wie Bis-diazobenzidine, oder
- photoaktive Cross-Linker, wie p-Azido-phenylglyoxal, p-Azidophenylisothiocyanat, p-Azido-phenacylbromid, 4,4'-Diazidostilben-2,2'-disulfonsäure-di-Natriumsalz, oder
- die Kopplung erfolgt via Diazotierungsreaktion, ausgehend von freien aromatischen NH₂-Gruppierungen an der Trägerverbindung.

Der Träger T₁ ist vorzugsweise das bereits oben erwähnte Cellulosederivat der allgemeinen Formel I oder ein anderes Cellulosederivat mit aromatischen Substituenten mit mindestens einer freien Aminogruppe. So sind beispielsweise Cellulosederivate wie p-Aminophenylcellulose, p-Aminobenzylcellulose, p-Aminobenzoylcellulose oder p-Phenylendiamin-substituierte Cellulosederivate anders als das 1,4-Phenylendiamin-Cellulosederivat der allgemeinen Formel I einsetzbar.

Des weiteren kann die Trägermatrix T₁ und/oder T₂ aber auch aus NH₂-haltigen Polymeren bestehen, wie Polyaminopolystyrole, Polyacrylamidderivate, oder makromolekularen Verbindungen anders als Cellulosederivate, beispielsweise Proteine, wie Collagene.

Die weiteren, nach dem "sandwich-Prinzip" angeknüpften Trägerverbindungen sind entweder mit dem Träger T₁ identisch oder von diesem verschieden. So kann beispielsweise T₃ auch eine (Transducer-)Unterlage, wie aminosilanisiertes, z.B. Aminopropyl-aktiviertes poröses Glas bzw. eine Faseroptik oder eine Silizium-Nitrid-Unterlage sein. Die erfindungsgemäßen Kopplungsstrukturen ermöglichen dabei eine - häufig angestrebte - kovalente Fixierung der Analyt-sensitiven Schicht an eine solche Unterlage.

Verknüpfungen mit weiteren, polymeren und/oder makromolekularen, NH₂-haltigen Trägerverbindungen der in Figur 1 allgemein dargestellten und oben beschriebenen Art sind auch dann eine bevorzugte Modifikation von Reagenz-Festphasen, wenn die Trägerverbindung erfindungsgemäß ein Cellulosederivat der allgemeinen Formel I ist und als Kopplungsstrukturen andere als die erfindungsgemäßen vorliegen. Als Kopplungsstruktur kommt in diesem Fall vorzugsweise Disulfonsäureamid in Betracht.

Reagenz-Festphasen, die als Trägerverbindung ein Cellulosederivat der allgemeinen Formel I enthalten, sind via NH₂-Gruppierungen der Trägerverbindung vorzugsweise weiterhin mit oxidativ kupplungsfähigen Verbindungen verknüpft. Bei den oxidativ kupplungsfähigen Verbindungen handelt es sich um sog. Kupplerverbindungen, wie sie aus der Farbfotografie bzw. aus der Patentliteratur zur Teststreifen-Entwicklung auf der Grundlage oxidativer Kupplungsreaktionen (Peroxidase-katalysiert) mit N,N-disubstituierten p-Phenylendiaminen bekannt sind. Die oben genannten erfindungsqemäßen Reagenz-Festphasen enthalten als oxidativ kupplungsfähige Verbindungen vorzugsweise Phenol oder Naphthol oder Derivate davon. Damit werden aromatische NH₂-Gruppierungen der Cellulosederivate unter oxidativen Kupplungsbedingungen in eine chinon-analoge Struktur überführt. Diese Ausgestaltung hat insbesondere den Vorteil breitgefächerter Modifizierungs- bzw. Einsatzmöglichkeiten der Redoxchromogenen Eigenschaften, insbesondere der Licht-Absorptions- und Redoxpotential-Eigenschaften und ihrer pH-Abhängigkeit. Sind oxidative Kupplungsstrukturen weiterhin mit Rezeptorverbindungen verknüpft, hat das zusätzlich durch eine supramolekülgeometrisch ausgezeichnete Kopplung von Analyt-affiner Rezeptorstruktur an die Redox-chromogene Kupplungsstruktur eine vorteilhafte Wirkung. Bei Verwendung von Cellulosederivaten der allgemeinen Formel I als Trägerverbindungen in Reagenz-Festphasen ist die Kopplungsstruktur vorzugsweise eine oxidativ kupplungsfähige Verbindung. Derartige Reagenz-Festphasen sind insbesondere für analytische Zwecke bei Redoxsystemen, insbesondere Wasserstoffperoxid-erzeugenden bzw. verbrauchenden Enzym/Substrat-Systemen oder Chinoprotein-Enzym/Substrat-Systemen einsetzbar.

Als Rezeptorverbindungen kommen Oxidoreduktase-Enzyme, Immunoproteine mit Oxidase- oder Peroxidase-Markern, Redox-Metallionen-Chelatverbindungen in Betracht, vorzugsweise Oxidoreduktase-Enzyme, insbesondere Flavoprotein-Oxidasen und/oder Peroxidase-Enzym, wenn die Trägerverbindung ein Cellulosederivat insbesondere der allgemeinen Formel I ist.

Derartige Reagenz-Festphasen sind hervorragend für den Einsatz bei der (bio)chemischen Sensor-Transducer-Entwicklung, vorzugsweise für Analyte auf dem Gebiet der medizinischen Diagnostik, der Umweltanalytik, der Lebensmittelanalytik oder der biotechnologischen Prozeßkontrolle oder bei anderen (bio)chemischen Analytikaufgaben oder bei präparativen Aufgabenstellungen, insbesondere in der Biotechnologie oder in der (bio)chemischen Praxis, geeignet.

Es ist aber auch denkbar, als Rezeptorverbindung nur Cofaktoren von Enzymen, wie FAD,NAD o.ä., einzusetzen, weil in Kombination mit den entsprechenden Enzymproteinen und bei Anwendung entsprechender supramolekularer Architekturvarianten nach Figur 1 ein vielfältiger Einsatz, insbesondere auf Gebieten der Analytik, in der Enzymtechnologie bzw. Biotechnologie, möglich ist.

Bei Einsatz der erfindungsgemäßen Reagenz-Festphasen treten die Rezeptorverbindungen mit einer affinen Verbindung zum Rezeptor in Wechselwirkung. Dies ist oft verbunden mit einer Umsetzungsreaktion, die durch eine in supramolekularer Nachbarschaft positionierte Indikator- bzw. Signalstruktur S (vgl. in Figur 1) z.B. durch Änderung der optischen bzw. Elektronen-Transfer-Eigenschaften der Reagenz-Festphase in Abhängigkeit von der Konzentration der affinen Verbindung vermessen wird.

Es wird weiterhin ein einfaches Verfahren zur Herstellung von (bio)chemischen Reagenz-Festphasen bereitgestellt, bei dem man wenigstens eine NH₂-haltige Träger-verbindung mit einer Kopplungsverbindung und anschließend mit einer NH₂-haltigen Rezeptorverbindung reagieren läßt, wobei als Kopplungsverbindung erfindungsgemäß Ascorbinsäure, Dehydroascorbinsäure oder eine diesen Substanzen strukturell ähnliche Verbindung eingesetzt wird. Es zeigte sich überraschenderweise, daß eine Lösung von Ascorbinsäure, Dehydroascorbinsäure oder 2,3-Diketogulonsäure bzw. strukturell ähnlichen Verbindungen als bifunktionelle Reagenzien unter kovalenter Kopplung bzw. Immobilisierung NH₂-haltiger Verbindungen reagiert.

Die Herstellung von (bio)chemischen Reagenz-Festphasen läßt sich nach dem erfindungsgemäßen Verfahren je nach Anzahl und Art bzw. Struktur der (Festphasen-)Schichtfolge gemäß der Reihenfolge von NH₂-haltigen polymeren oder makromolekularen Trägerverbindungen, Rezeptorverbindungen und/oder Indikator- bzw. Signalstrukturen sowie bezüglich der Art der Kopplungsreaktion(en) nach vielfältigen Vorgehensweisen gestalten:

Sollen beispielsweise nach dem erfindungsgemäßen Verfahren mehrere NH₂-haltige Verbindungen nach dem oben erwähnten "sandwich-Prinzip" verknüpft werden, läßt man die NH₂-reaktive Kopplungsverbindung, beispielsweise Ascorbinsäure, vorzugsweise in Dimethylacetamid, Dimethylsulfoxid oder Tetrahydrofuran gelöst, auf die NH₂-haltige Trägerverbindung bei Raumtemperatur oder, falls erforderlich, bei +4°C einwirken. Nach einer Einwirkzeit, die je nach Reaktivität der Reaktionspartner zwischen einigen Minuten und einigen Stunden liegen kann, wird nicht umgesetzte Kopplungsverbindung mit bidest.

Wasser oder Alkohol abgewaschen. Anschließend wird die funktionalisierte Trägerverbindung mit der anzukoppelnden NH₂-haltigen Verbindung, wie Rezeptorverbindung und/oder Cellulosederivat mit aromatischen NH₂-haltigen Substituenten, insbesondere der oben genannten Formel I, behandelt. Für diese Behandlung liegt die Rezeptorverbindung in der Regel in wässriger Lösung und das Cellulosederivat insbesondere in Dimethylacetamid-Lösung vor.

Danach kann sich bei Verwendung des genannten Cellulosderivats als Trägerverbindung zusätzlich noch eine Reaktion mit einem Reduktionsmittel, vorzugsweise mit Natrium-cyanoborhydrid (NaBH₃CN), anschließen; oder es erfolgt erneut eine Reaktion mit einer der genannten Kopplungsverbindungen und/oder eine Behandlung mit einer oxidativ kupplungsfähigen Verbindung, insbesondere mit Phenol, Naphthol oder Derivaten davon. Als Phenolderivat kommmt vorzugsweise eine wässrige (Puffer-) Lösung (pH 7 bis pH 8) von 2,4,6-Tribrom-3-hydroxybenzoesäure, Wasserstoffperoxid und Peroxidase-Enzym als Katalysator zum Einsatz. Dabei erfolgt die oxidative Kupplungsreaktion an den freien aromatischen NH₂-Gruppierungen des 1,4-Phenylendiamin-Restes bei Raumtemperatur oder - falls erforderlich - bei +4°C in 10 bis 60 Minuten, wie aus der biochemischen, Peroxidasekatalysierten Analytik bekannt ist.

Danach wird die Festphase, die jetzt ausgehend von dem oben genannten Phenolderivat blaugefärbte chinonanaloge Strukturen als oxidatives Kupplungsprodukt enthält, mit Wasser gewaschen. Anschließend kann die Reagenz-Festphase je nach Anwendungszweck eingesetzt werden, oder sie wird erneut mit einer NH₂-haltigen Verbindung zwecks Kopplungsreaktion an den chinon-analogen Strukturen behandelt. Als NH₂-haltige Verbindung wird eine Trägerverbindung, vorzugsweise ein Cellulosederivat nach Formel I, und/oder eine Rezeptorverbindung, vorzugsweise Oxidoreduktase-Enzyme, insbesondere Flavoprotein-Oxidasen in Konzentrationen von 10 bis 1000 U/ml wässriger Lösung, eingesetzt.

Anschließend kann die Reagenz-Festphase je nach Anwendungszweck eingesetzt werden oder erneut mit einer erfindungsgemäßen oder bekannten NH₂-reaktiven Kopplungsverbindung und danach mit einer Trägerverbindung und/oder einer Rezeptorverbindung und/oder einer Indikatorverbindung, vorzugsweise einem Redox-Chromogen, wie einem oxidativen Kupplungsprodukt oder einer Redox-Metallionen-Chelatverbindung, erforderlichenfalls noch mehrmals behandelt werden, um (bio)chemische Reagenz-Festphasen für die oben genannten Anwendungszwecke bereit zu stellen.

Der wesentliche Vorteil des erfindungsgemäßen Verfahrens besteht darin, daß es neben der einfachen Durchführung unter milden Reaktionsbedingungen, insbesondere durch Einsatz eines Cellulosederivats nach Formel I, vielfältige Modifizierungsmöglichkeiten im Hinblick auf den Anwendungszweck gestattet, und zwar durch große Variabilität der Kopplungsreaktionen und der mehrfachen Schichtfolge bezüglich Cellulosederivat bzw. Trägerverbindung, Rezeptorverbindung und/oder Indikatorverbindung bis zum Erreichen einer hohen Beladungsdichte mit Erkennungs- und Signalstrukturen und ihrer Anpassung an den Anwendungszweck. Darüber hinaus erweist sich auch als vorteilhaft, daß bei Anwendung der erfindungsgemäßen Kopplungsreaktionen die eingesetzte Ascorbinsäure bzw. Dehydroascorbinsäure leicht verfügbar und untoxisch ist.

### Ausführungsbeispiele:

### 1.1 Herstellung erfindungsgemäßer Festphasen

A) Schichten aus 1,4-Phenylendiamin-Cellulosederivaten
   - Bereitung der Schichten:
      Es werden Glasstäbe (d = 1,3 mm) in eine vikose Lösung eines 1,4-Phenylendiamin-Cellulosederivates (vgl. Patentanmeldung Nr. : "Neue aromatische Amin-Cellulosederivate und Verfahren zu deren Herstellung") in DMAc (50 bis 150 mg Cellulosederivat/ml DMAc) eingetaucht (ca. 4 mm Tauchhöhe), nach kurzer Zeit (1 bis 2 Minuten) herausgenommen und für 5 bis 10 Stunden an der Luft getrocknet. Danach befinden sich feste und transparente Schichten an den Glasstäben, die bei den folgenden Synthesebeispielen eingesetzt werden.
A1) Glucose-sensitive Cellulose-Festphase (optisches Signal bei 550 nm)
   - Funktionalisierung der Festphase mittels Ascorbinsäure-Lösung
      Ein nach Verfahrensweise A) vorbereiteter Glasstab wird mit der Cellulosederivat-Schicht in eine Lösung von 1 g Ascorbinsäure in 2,5 ml DMAc eingetaucht, nach kurzer Einwirkzeit (ca. 1 Minute) herausgenommen und anschließend für 4 bis 5 Stunden an der Luft getrocknet. Danach wird die inzwischen purpur gefärbte Festphase ca. 5 mal mit bidest. Wasser gewaschen und kann dann zur Immobilisierung einer Rezeptorverbindung eingesetzt werden:
   - Enzym-Immobilisierung an der Festphase
      Die funktionalisierte Festphase wird in eine Enzymlösung von Glucose-Oxidase (GOD) in bidest. Wasser (40 U GOD/ml Wasser) eingetaucht und für 8 bis 15 Stunden bei 4°C aufbewahrt.
      Anschließend wird die purpur gefärbte Festphase 3 bis 5 mal mit bidest. Wasser gewaschen und danach mit einer wässrigen Ascorbinsäure-Lösung kurzzeitig (ca. 1 Minute) behandelt, um die Festphase in die Leuko-Form zu überführen. Die Festphase spricht bei einer Wellenlänge von 540...560 nm auf Glukosekonzentrationen an.
A2) Glutamat-sensitive Cellulose-Festphase (optisches Signal bei 550 nm)
   Eine nach Verfahrensweise A1) funktionalisierte Festphase wird in eine Enzymlösung von Glutamat-Oxidase (GlOD) in bidest. Wasser (15 U GlOD/ml Wasser) eingetaucht und für 8 bis 15 Stunden bei 4°C aufbewahrt. Anschließend wird die purpur gefärbte Festphase ca. 5 mal mit bidest. Wasser gewaschen und danach mit einer wässrigen Ascorbinsäure-Lösung kurzzeitig (ca. 1 Minute) behandelt, um die Festphase in die Leuko-Form zu überführen. Die Festphase spricht bei einer Wellenlänge von 540...560 nm auf Glutamatkonzentrationen an.
A3) Glucose-sensitive Cellulose-Festphase (optisches Signal bei 660 nm)
   Eine Glucose-sensitive Festphase, bereitet nach Verfahrensweise A1, wird mit einer Lösung aus 2ml Pufferlösung (pH 8), 1 ml einer 7,5 mmolaren 2,4,6-Tribrom-3-hydroxybenzoesäure-Lösung in bidest. Wasser, 100 ul einer 100 mmolaren H₂O₂-Lösung und 50 ul einer Peroxidase(POD)-Lösung in bidest. Wasser (150 U POD/ml) behandelt. Nach 30 bis 60 Minuten Stehen bei Raumtemperatur wird die inzwischen blau gefärbte Festphase aus der Lösung herausgenommen, mit bidest. Wasser ca. 5 mal gewaschen, anschließend kurzzeitig (ca. 1 Minute) mit einer wäßrigen Ascorbinsäure-Lösung behandelt und nochmals ca. 5 mal mit bidest. Wasser gewaschen. Die Festphase spricht auf Glucose-Konzentrationen bei einer Wellenlänge von 650...670 nm an.
A4) Glucose-sensitive Cellulose-Festphase (optisches Signal bei 660 nm)
   Ein beschichteter Glasstab, bereitet nach Verfahrensweise A), wird kurzzeitig (ca. 2 Sekunden) in DMAc eingetaucht und anschließend mit bidest. Wasser ca. 3 mal gewaschen. Danach wird die Festphase mit einer Lösung, wie unter Beispiel A3) beschrieben, behandelt. Nach ca. 30 Minuten wird die dunkelblau gefärbte Festphase mit bidest. Wasser ca. 5 mal gewaschen und anschließend mit einer GOD-Lösung, wie bei Beispiel A1) beschrieben, behandelt bzw. aufgearbeitet. Die Festphase spricht nach Überführung in die Leuko-Form mittels Ascorbinsäure-Lösung (vgl. unter A3) auf Glukose-Konzentrationen bei einer Wellenlänge um 660 nm an.
A5) Glucose-sensitive Cellulose-Festphase (optisches Signal bei 550 nm)
   - Funktionalisierung der Festphase mittels Benzol-1,3-disulfonsäuredichlorid
      Ein beschichteter Glasstab, bereitet nach Verfahrensweise A), wird in eine Lösung aus 500 mg Benzol-1,3-disulfonsäuredichlorid in 2 ml DMAc eingetaucht, nach ca. 1 Minute herausgenommen und anschließend für ca. 5 Stunden an der Luft getrocknet. Danach wird die inzwischen rot gefärbte Festphase ca. 5 mal abwechselnd mit Äthanol und bidest. Wasser gewaschen und danach mit einer Enzym-Lösung, z.B. GOD-Lösung wie bei Beispiel A1) beschrieben, behandelt bzw. aufgearbeitet. Die Festphase spricht im Falle GOD als Enzymbeispiel auf Glucose-Konzentrationen bei einer Wellenlänge um 550 nm an.

### 1.2 Beispiel einer erfindungsgemäßen Festphase

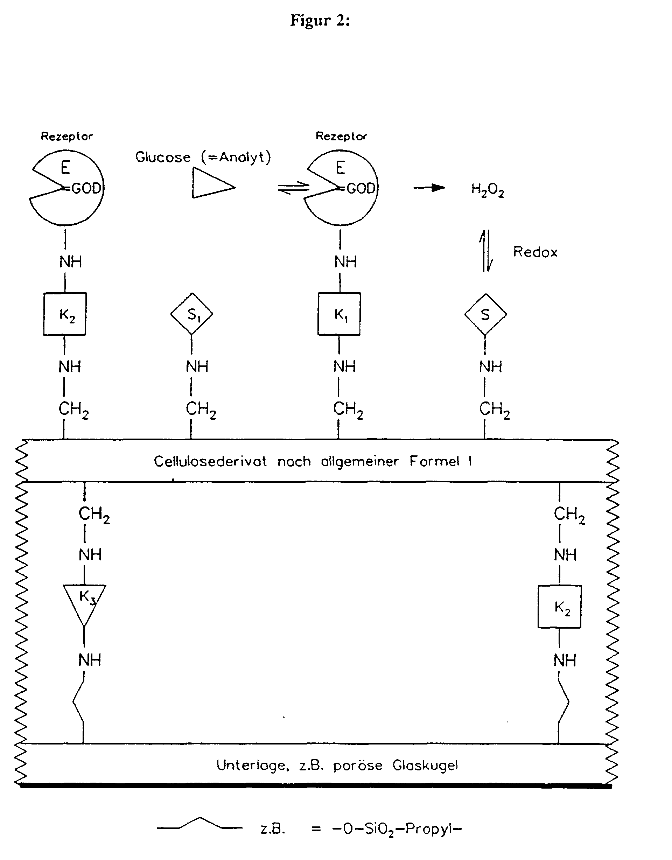

Ein Beispiel einer erfindungsgemäßen Festphase ist in Figur 2 dargestellt und wird im folgenden näher erläutert:

Die (bio)chemische Reagenz-Festphase gemäß Figur 2 enthält als Unterlage Aminopropyl-aktiviertes poröses Glas, das durch Ascorbinsäure als Kopplungsstrukturen K₂ und K₃ via 1,4-Phenylendiamin-Reste mit einem Cellulosederivat der allgemeinen Formel I verknüpft ist.

An das Cellulosederivat ist via 1,4-Phenylendiamin-Reste durch die chinon-analoge Kopplungsstruktur K₁ und zusätzlich durch Ascorbinsäure K₂ Glucoseoxidase (GOD) als Rezeptor immobilisiert.

Als (Redox-) Indikator- bzw. Signalstrukturen liegen freie 1,4-Phenylendiamin-Reste S 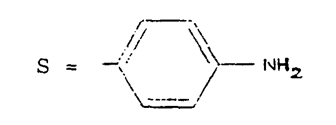 und zusätzlich mittels Ascorbinsäure reduzierte oxidative Kupplungsstrukturen S₁ vor, die ausgehend von 2,4,6-Tribrom-3-hydroxybenzoesäure als kupplungsfähige Verbindung angekoppelt und nicht durch GOD besetzt sind. Darüber hinaus stellt K₁ ein Redox-Chromogen dar.

Glucose (=Analyt) wird als affines Enzym-Substrat in ein entsprechendes Produkt unter Erzeugung von Wasserstoffperoxid (H₂O₂) umgesetzt. Das entstehende H₂O₂ reagiert mit einer supramolekülgeometrisch benachbarten Leuko-Form der Redox-Indikatorstruktur S unter Bildung der chromogenen Ox.-Form von S. Dabei tritt eine Änderung der spektrophotometrischen Eigenschaften der Reagenz-Festphase ein, die über einen optischen Wellenleiter, wie eine Faseroptik, durch Anwendung einer optischen Detektion, beispielsweise mittels einer DIN-Diode, vermeßbar ist.

## Patentansprüche

1. (Bio)chemische Reagenz-Festphasen, bestehend aus einer polymeren oder makromolekularen, NH₂-haltigen Trägerverbindung, an die Kopplungsstrukturen über NH₂-Gruppen kovalent gebunden sind, die weiterhin mit Rezeptor-und/oder Indikatorverbindungen verknüpft sind und die somit die Rezeptor- und/oder Indikatorverbindungen an den Träger koppeln,
**dadurch gekennzeichnet,**
daß die Kopplungsstrukturen aus Ascorbinsäure oder Dehydroascorbinsäure oder einer diesen Substanzen strukturell ähnlichen Verbindung bestehen.

2. Reagenz-Festphasen nach Anspruch 1,
**dadurch gekennzeichnet,**
daß die strukturell ähnliche Verbindung L-2,3-Diketogulonsäure oder ein Derivat davon ist.

3. Reagenz-Festphasen nach Anspruch 1 oder 2,
**dadurch gekennzeichnet,**
daß über mindestens einer Kopplungsstruktur wenigstens eine weitere, polymere und/oder makromolekulare, NH₂-haltige Trägerverbindung nach dem "sandwich-Prinzip" geknüpft ist.

4. Reagenz-Festphasen nach Anspruch 3,
**dadurch gekennzeichnet,**
daß an NH2-Gruppen jeder weiteren, polymeren und/oder makromolekularen Trägerverbindung Rezeptorverbindungen durch Kopplungsstrukturen fixiert sind.

5. Reagenz-Festphasen nach einem der vorhergehenden Ansprüche,
**dadurch gekennzeichnet,**
daß die Rezeptorverbindungen weiterhin mit einer affinen Verbindung zum Rezeptor in Wechselwirkungskontakt vorliegen.

6. Reagenz-Festphasen nach einem der vorhergehenden Ansprüche,
**dadurch gekennzeichnet,**
daß die NH₂-haltige Trägerverbindung ein Cellulosederivat mit aromatischen Substituenten ist, die mindestens eine freie Aminogruppe enthalten.

7. Reagenz-Festphasen nach Anspruch 6,
**dadurch gekennzeichnet,**
daß das Cellulosederivat eine Verbindung der folgenden, allgemeinen Formel I ist: 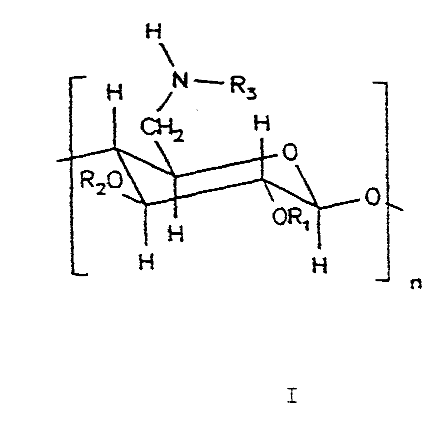 in der
R₁, R₂ = H
oder R₁ = H, R₂ = Substituent mit einem Substitutionsgrad < 1
oder R₁ = Substituent mit einem Substitutionsgrad ≤ 1, R₂ = H
oder R₁, R₂ = Substituent mit einem Substitutionsgrad ≤ 2
und R₃ ein aromatischer Substituent, der mindestens eine freie Aminogruppe enthält und einem Substitutionsgrad ≤ 1 bedeutet.

8. Reagenz-Festphasen nach Anspruch 7,
**dadurch gekennzeichnet,**
daß R₃ ein Rest der Formel 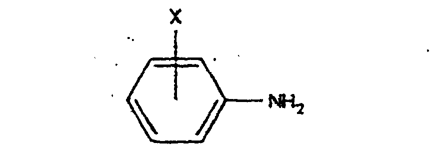 X= H oder H und/oder Substituenten bedeutet.

9. Reagenz-Festphasen nach Anspruch 7 oder 8,
**dadurch gekennzeichnet,**
daß der Substituent R₁ und/oder R₂ ein Akyl, vorzugsweise CH₃, oder ein Acyl, vorzugsweise Acetyl, oder ein Tosylrest oder ein Tresylrest ist.

10. Reagenz-Festphasen nach einem der Ansprüche 6 bis 9,
**dadurch gekennzeichnet,**
daß NH₂-Gruppen der Trägerverbindung mit oxidativ kupplungsfähigen Verbindungen verknüpft sind.

11. Reagenz-Festphasen nach Anspruch 10,
**dadurch gekennzeichnet,**
daß die oxidativ kupplungsfähige Verbindung ein Phenol oder Naphthol oder ein Phenol- oder Naphtholderivat ist.

12. Reagenz-Festphasen nach Anspruch 10 oder 11,
**dadurch gekennzeichnet,**
daß oxidative Kupplungsstrukuren weiterhin mit Rezeptorverbindungen verknüpft sind.

13. Reagenz-Festphasen nach einem der Ansprüche 6 bis 12,
**dadurch gekennzeichnet,**
daß die Rezeptorverbindung ein Oxidoreduktase-Enzym, vorzugsweise Flavoprotein-Oxidase, ist.

14. Verfahren zur Herstellung von (bio)chemischen Reagenz-Festphasen gemäß einem der Ansprüche 1 bis 13, bei dem man wenigstens eine NH₂-haltige Trägerverbindung mit einer Kopplungsverbindung und anschließend mit einer Rezeptorverbindung reagieren läßt
**dadurch gekennzeichnet,**
daß als Kopplungsverbindung Ascorbinsäure, Dehydroascorbinsäure oder eine diesen Substanzen strukturell ähnliche Verbindung eingesetzt wird.

15. Verfahren nach Anspruch 14,
**dadurch gekennzeichnet,**
daß als strukturell ähnliche Verbindung L-2,3-Diketogulonsäure oder ein Derivat davon eingesetzt wird.

16. Verfahren nach Anspruch 14 oder 15,
**dadurch gekennzeichnet,**
daß als Trägerverbindung ein Cellulosederivat mit aromatischen Substituenten, die mindestens eine freie Aminogruppe enthalten, eingesetzt wird.

17. Verfahren nach Anspruch 16,
**dadurch gekennzeichnet,**
daß als Cellulosederivat eine Verbindung der allgemeinen Formel I gemäß einem der Ansprüche 7 bis 9 eingesetzt wird.

18. Verfahren nach Anspruch 16 oder 17,
**dadurch gekennzeichnet,**
daß man die Trägerverbindung nach Reaktion mit der Kopplungsverbindung und der Rezeptorverbindung zusätzlich noch mit einem Reduktionsmittel, vorzugsweise mit Natriumcyanoborhydrid (NaBH₃CN), reagieren läßt.

19. Verfahren nach Anspruch 16 oder 17,
**dadurch gekennzeichnet,**
daß man die Trägerverbindung nach Reaktion mit der Kopplungsverbindung und der Rezeptorverbindung zusätzlich noch mit einer oxidativ kupplungsfähigen Verbindung reagieren läßt.

20. Verfahren nach Anspruch 19,
**dadurch gekennzeichnet,**
daß man die Reaktion mit der oxidativ kupplungsfähigen Verbindung mit Wasserstoffperoxid und Peroxidase katalysiert ablaufen läßt.

21. Verfahren nach Anspruch 19 oder 20,
**dadurch gekennzeichnet,**
daß man die Trägerverbindung nach Reaktion mit der oxidativ kupplungsfähigen Verbindung zusätzlich mit einer Rezeptorverbindung reagieren läßt.

22. Verfahren nach einem der Ansprüche 19 bis 21,
**dadurch gekennzeichnet,**
daß als oxidativ kupplungsfähige Verbindung ein Phenol oder Naphthol oder ein Phenol- oder Naphtholderivat eingesetzt wird.

23. Verfahren nach Anspruch 22,
**dadurch gekennzeichnet,**
daß 2,4,6-Tribrom-3-hydroxy-benzoesäure als Phenolderivat eingesetzt wird.

24. Verfahren nach einem der Ansprüche 16 bis 23,
**dadurch gekennzeichnet,**
daß als Rezeptorverbindung ein Oxidoreduktase-Enzym, vorzugsweise Flavoprotein-Oxidase, eingesetzt wird.

25. Verwendung von Reagenz-Festphasen nach einem der Ansprüche 1 bis 13 als analyt-sensitive Festphasen in der Sensorik.

26. Verwendung von Reagenz-Festphasen nach einem der Ansprüche 1 bis 13 in Bioreaktoren.

## Revendications

1. Phases solides de réactifs (bio)chimiques, formées d'un composé de support polymère ou macromoléculaire contenant des groupes NH₂, auquel sont liées des structures de couplage par covalence au moyen de groupes NH₂, qui sont reliées en outre à des composés récepteurs et/ou indicateurs et qui couplent ainsi les composés récepteurs et/ou indicateurs avec le support, caractérisées en ce que les structures de couplage sont formées d'acide ascorbique ou d'acide déhydro-ascorbique ou d'un composé analogue à ces substances du point de vue structurel.

2. Phases solides de réactifs selon la revendication 1,
caractérisées en ce que le composé analogue du point de vue structurel est l'acide L-2,3-dicétogulonique ou un dérivé de celui-ci.

3. Phases solides de réactifs selon la revendication 1 ou 2, caractérisées en ce qu'au moins un composé de support polymère et/ou macromoléculaire supplémentaire contenant des groupes NH₂ est lié au moyen d'au moins une structure de couplage selon le "principe sandwich".

4. Phases solides de réactifs selon la revendication 3, caraetérisées en ce que des composés récepteurs sont fixés à des groupes NH₂ de chaque composé de support polymère et/ou macromoléculaire supplémentaire au moyen de structures de couplage.

5. Phases solides de réactifs selon l'une des revendications précédentes, caractérisées en ce que les composés récepteurs se présentent en outre avec une liaison affine pour le récepteur par contact interactif.

6. Phases solides de réactifs selon l'une des revendications précédentes, caractérisées en ce que le composé de support contenant des groupes NH₂ est un dérivé cellulosique avec des substituants aromatiques qui contiennent au moins un groupe amino libre.

7. Phases solides de réactifs selon la revendication 6, caractérisées en ce que le dérivé cellulosique est un composé répondant à la formule générale I suivante : 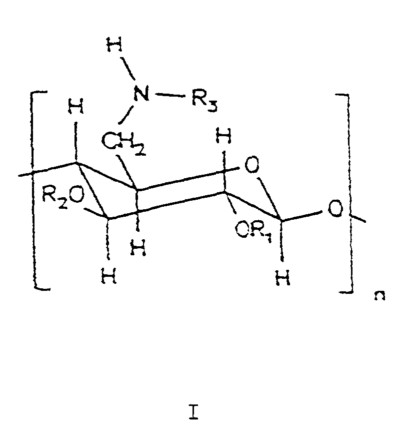 dans laquelle
R₁, R₂ = H
ou R₁ = H, R₂ = un substituant avec un taux de substitution ≤ 1
ou R₁ = un substituant avec un taux de substitution ≤ 1, R₂ = H
ou R₁, R₂ = un substituant avec un taux de substitution ≤ 2
et R₃ signifie un substituant aromatique qui contient au moins un groupe amino libre et a un taux de substitution ≤ 1.

8. Phases solides de réactifs selon la revendication 7, caractérisées en ce que R₃ signifie un radical de formule 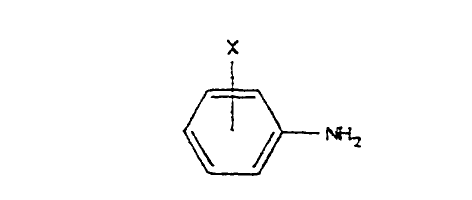 X = H ou H et/ou des substituants

9. Phases solides de réactifs selon la revendication 7 ou 8, caractérisées en ce que le substituant R₁ et/ou R₂ est un radical alkyle, de préférence CH₃, ou un radical acyle, de préférence acétyle, ou un radical tosyle ou un radical trésyle.

10. Phases solides de réactifs selon l'une des revendications 6 à 9, caractérisées en ce que des groupes NH₂ du composé de support sont liés à des composés pouvant être couplés par oxydation.

11. Phases solides de réactifs selon la revendication 10, earactérisées en ce que composé pouvant être couplé par oxydation est un phénol ou un naphtol ou un dérivé de phénol ou de naphtol.

12. Phases solides de réactifs selon la revendication 10 ou 11, caractérisées en ce que les structures de couplage par oxydation sont liées en outre à des composés récepteurs.

13. Phases solides de réactifs selon l'une des revendications 6 à 12, caractérisées en ce que le composé récepteur est une enzyme oxydoréductase, de préférence une flavoprotéine-oxydase.

14. Procédé de préparation de phases solides de réactifs (bio)chimiques selon l'une des revendications 1 à 13, dans lequel on fait réagir au moins un composé de support contenant des groupes NH₂ avec un composé de couplage et ensuite avec un composé récepteur, caractérisé en ce qu'on utilise, comme composé de couplage, l'acide ascorbique, l'acide déhydro-ascorbique ou un composé analogue à ces substances du point de vue structurel.

15. Procédé selon la revendication 14, caractérisé en ce qu'on utilise, comme composé analogue du point de vue structurel, de l'acide L-2,3-dicétogulonique ou un dérivé de celui-ci.

16. Procédé selon la revendication 14 ou 15, caractérisé en ce qu'on utilise, comme composé de support, un dérivé cellulosique avec des substituants aromatiques qui contiennent au moins un groupe amino libre.

17. Composé selon la revendication 16, caractérisé en ce qu'on utilise, comme dérivé cellulosique, un composé de formule générale I selon l'une des revendications 7 à 9.

18. Procédé selon la revendication 16 ou 17, caractérisé en ce que, après la réaction avec le composé de couplage et le composé récepteur, on fait aussi réagir le composé de support en outre avec un agent réducteur, de préférence avec du cyanoborohydrure de sodium (NaBH₃CN).

19. Procédé selon la revendication 16 ou 17, caractérisé en ce que, après la réaction avec le composé de couplage et le composé récepteur, on fait aussi réagir le composé de support en outre avec un composé pouvant être couplé par oxydation.

20. Procédé selon la revendication 19, caractérisé en ce qu'on effectue la réaction avec le composé pouvant être couplé par oxydation en la catalysant avec du peroxyde d'hydrogène et une peroxydase.

21. Procédé selon la revendication 19 ou 20, caractérisé en ce que, après la réaction avec le composé pouvant être couplé par oxydation, on fait réagir le composé de support en outre avec un composé récepteur.

22. Procédé selon l'une des revendications 19 à 21, caractérisé en ce qu'on utilise, comme composé pouvant être couplé par oxydation, un phénol ou un naphtol ou un dérivé de phénol ou de naphtol.

23. Procédé selon la revendication 22, caractérisé en ce qu'on utilise de l'acide 2,4,6-tribromo-3-hydroxybenzoïque en tant que dérivé phénolique.

24. Procédé selon l'une des revendications 16 à 23, caractérisé en ce qu'on utilise, comme composé récepteur, une enzyme oxydoréductase, de préférence de la flavoprotéine-oxydase.

25. Utilisation de phases solides de réactifs selon l'une des revendications 1 à 13 en tant que phases solides sensibles à un analyte dans une analyse sensorielle.

26. Utilisation de phases solides de réactifs selon l'une des revendications 1 à 13 dans des bioréacteurs.

## Claims

1. Bio)chemical reagent solid phases comprising a polymer or macro-molecular NH₂ containing carrier compound to which coupling structures are covalently bound via NH₂ groups, and which are moreover linked to receptor and/or indicator compounds, thus coupling the receptor and/or indicator compounds to the carrier, **characterised in that** the coupling structures are composed of ascorbic acid or dehydro-ascorbic acid or a compound which is structurally similar to one of these substances.

2. Reagent solid phases according to Claim 1, **characterised in that** the structurally similar compound is L-2,3-diketo-gulonic acid or a derivative thereof.

3. Reagent solid phases according to Claim 1 or 2, **characterised in that** via at least one coupling structure is linked at least one further polymer and/or macro-molecular NH₂ containing carrier compound by the "sandwich principle".

4. Reagent solid phases according to Claim 3, **characterised in that** to NH₂ groups of each additional polymer and/or macro-molecular carrier compound are fixed receptor compounds by way of coupling structures.

5. Reagent solid phases according to one of the above claims, **characterised in that** the receptor compounds are moreover present with a compound having affinity for the receptor in interactive contact.

6. Reagent solid phases according to one of the above claims, **characterised in that** the NH₂ containing carrier compound is a cellulose derivative with aromatic substituents containing at least one free amino group.

7. Reagent solid phases according to Claim 6, **characterised in that** the cellulose derivative is a compound of the following general formula 1: 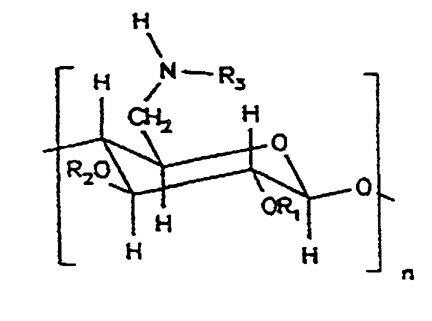 where
R₁, R₂ =
or R₁ = H, R₂ = substituent with a substitution degree ≤ 1
or R₁ = substituent with a substitution degree ≤ 1, R₂ = H
or R₁, R₂ = substituent with a substitution degree ≤ 2
and R₃ an aromatic substituent containing at least one free amino group and a substitution degree ≤ 1.

8. Reagent solid phases according to Claim 7, **characterised in that** R₃ is a residue of the formula 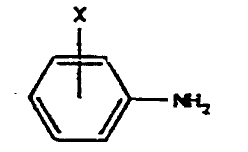 where X = H or H and/or substituents.

9. Reagent solid phases according to Claim 7 or 8, **characterised in that** the substituent R₁ and or R₂ is an alkyl, preferably CH₃, or an acyl, preferably acetyl, or a tosyl residue or a tresyl residue.

10. Reagent solid phases according to one of Claims 6 to 9, **characterised in that** NH₂ groups of the carrier compound are linked with compounds which can be oxidatively coupled.

11. Reagent solid phases according to Claim 10, **characterised in that** the compound which can be oxidatively coupled is a phenol or naphthol or a phenol or naphthol derivative.

12. Reagent solid phases according to Claim 10 or 11, **characterised in that** oxidative coupling structures are also linked to receptor compounds.

13. Reagent solid phases according to one of Claims 6 to 12, **characterised in that** the receptor compound is an oxido-reductase enzyme, preferably Flavoprotein-oxidase.

14. Process for production of (bio)chemical reagent solid phases according to one of Claims 1 to 13 in which at least one NH₂ containing carrier compound is allowed to react with a coupling compound and thereafter with a receptor compound, **characterised in that** ascorbic acid, dehydro-ascorbic acid or a compound structurally similar to the above is used.

15. Process according to Claim 14, **characterised in that** L-2,3-diketogulonic acid or a derivative thereof is used.

16. Process according to Claim 14 or 15, **characterised in that** a cellulose derivative with aromatic substituents which contain at least one free amino group is used.

17. Process according to Claim 16, **characterised in that** a compound of the general formula I according to one of Claims 7 to 9 is used as cellulose derivative.

18. Process according to Claim 16 or 17, **characterised in that,** following reaction with the coupling compound and the receptor compound, the carrier compound is additionally allowed to react with a reducing agent, preferably with sodium cyanoborohydride (NaBH₃CN).

19. Process according to Claim 16 or 17, **characterised in that,** following reaction with the coupling compound and the receptor compound, the carrier compound is additionally allowed to react with a compound which can be oxidatively coupled.

20. Process according to Claim 19, **characterised in that** the reaction with the compound which can be oxidatively coupled is allowed to proceed catalysed with hydrogen peroxide and peroxidase.

21. Process according to Claim 19 or 20, **characterised in that,** following reaction with the compound which can be oxidatively coupled, the carrier compound is additionally allowed to react with a receptor compound.

22. Process according to one of Claims 19 to 21, **characterised in that** a phenol or naphthol or a phenol or naphthol derivative is used as compound which can be oxidatively coupled.

23. Process according to Claim 22, **characterised in that** 2, 4, 6-tribromo-3-hydroxy-benzoic acid is used as phenol derivative.

24. Process according to one of Claims 16 to 23, **characterised in that** an oxidoreductase enzyme, preferably Flavoprotein-oxidase, is used as receptor compound.

25. Use of reagent solid phases according to one of Claims 1 to 13 as analyte-sensitive solid phases in the field of sensors.

26. Use of reagent solid phases according to one of Claims 1 to 13 in bio-reactors.
